# EUROPEAN PATENT SPECIFICATION

(11) **EP 2 720 670 B1**
(45) Date of publication and mention of the grant of the patent: **28.10.2015**
(21) Application number: 12729082.3
(22) Date of filing: 08.06.2012
(51) Int. Cl.: A61K 8/34, A61K 8/44, A61K 8/46, A61K 8/73, A61K 8/86, A61Q 5/02, A61Q 5/12, A61Q 19/10, A61K 8/84

(54) **Cosmetic composition comprising an anionic surfactant, a nonionic or amphoteric surfactant and a solid fatty alcohol, and cosmetic treatment process**
Kosmetische Zusammensetzung mit einem anionischen Tensid, einem nichtionischen oder amphoteren Tensid und einem festen Fettalkohol sowie kosmetisches Behandlungsverfahren
Composition cosmétique comprenant un tensioactif anionique, un tensioactif non ionique ou amphotère et un alcool gras solide, et procédé de traitement cosmétique

(30) Priority: 17.06.2011 FR 1155314; 31.07.2011 US 201161513605 P
(43) Date of publication of application: 23.04.2014
(73) Proprietor: L'OREAL, 75008 Paris (FR)
(72) Inventor: MATHONNEAU, Estelle, 93400 SAINT-OUEN (FR)
(74) Representative: Dodin, Catherine
(86) International application number: PCT/EP2012/060862
(87) International publication number: WO 2012/171850

(56) References cited:
- EP-A1- 1 025 833
- EP-A1- 1 120 104
- EP-A1- 1 380 284
- WO-A1-98/25579
- WO-A1-2009/079995
- DE-C1- 19 539 429
- FR-A1- 2 798 846
- FR-A1- 2 815 255
- US-A1- 2001 009 909
- US-A1- 2009 005 460
- "Sasol Olefins and Surfactants", Sasol , XP002670809, Retrieved from the Internet: URL:http://www.innovadex.com/documents/993 378.pdf?bs=2767&b=87088&st=20 [retrieved on 2011-03-05]

## Description

The present invention relates to a composition for washing and conditioning keratin materials, in particular keratin fibres, comprising one or more solid fatty alcohols, one or more first surfactants chosen from anionic surfactants, one or more second surfactants chosen from nonionic surfactants, and amphoteric or zwitterionic surfactants, and to the use of the said composition for washing and conditioning keratin materials, in particular keratin fibres, and also to a process using the said composition.

In the field of conditioning shampoos, a washing base is generally combined with a conditioning agent, which may be a cationic polymer, an amphoteric polymer, a silicone, a synthetic or natural oil, a fatty substance or a mixture thereof. These conditioning agents are used for improving the disentangling and softness of wet and dried hair, but may have a tendency to make the hair lank and dull.

The use of insoluble conditioning agents is greatly limited:
- firstly due to the stabilization difficulties in detergent compositions, if it is desired to maintain the level of the working qualities (stability, latherability, start of lathering),
- secondly due to cosmetic defects in terms of the lank, charging and regreasing effect associated with coarse or heterogeneous dispersions in these same detergent compositions.

Insoluble conditioning agents, in particular fatty alcohols, are known and used in shampoo compositions, especially in documents JP2002-20791, JP9-30938, US 2009/005 449 and US 2009/005 460.

The document WO9825579 is also relevant in this context.

However, these compositions do not have high-quality cosmetic performance especially in terms of disentangling and smoothing, and while maintaining the level of the working qualities.

Thus, there is still a need to improve the cosmetic properties of washing compositions without having an impact on the lathering properties of the said compositions, or, even, while improving them.

The Applicant has now discovered that the use of a combination of particular surfactants and solid fatty alcohols it is possible to overcome the drawbacks indicated above, and thus to obtain a composition that has improved cosmetic properties, in particular the smoothness, softness, suppleness and sheen.

One subject of the invention is thus a composition, especially for washing or cleansing keratin materials, in particular keratin fibres, comprising:
a) one or more anionic surfactants,
b) one or more surfactants chosen from nonionic surfactants, and amphoteric or zwitterionic surfactants, and
c) one or more solid and branched fatty alcohols containing more than 25 carbon atoms.

Another subject of the invention is the use of the said composition for washing, cleansing and conditioning keratin materials, in particular keratin fibres and more particularly the hair.

A subject of the invention is also a process for washing or cleansing and conditioning keratin fibres using the composition according to the invention.

Other subjects, characteristics, aspects and advantages of the invention will emerge even more clearly on reading the description and the various examples that follow.

In the present patent application, a species is termed as being "anionic" when it bears at least one permanent negative charge or when it can be ionized as a negatively charged species, under the conditions of use of the compositions of the invention (for example the medium or the pH) and not comprising any cationic filler.

In the present patent application, a species is termed as being "cationic" when it bears at least one permanent positive charge or when it can be ionized as a positively charged species, under the conditions of use of the compositions of the invention (for example the medium or the pH) and not comprising any anionic filler.

A species is termed as being "nonionic" when it is neither cationic nor anionic within the meaning of the present patent application, in particular when it comprises no cationic or anionic groups within the meaning of the present patent application.

According to the present invention, the composition comprises one or more anionic surfactants.

The term "anionic surfactant" means a surfactant comprising, as ionic or ionizable groups, only anionic groups. These anionic groups are preferably chosen from-CO₂H, -CO₂⁻, -SO₃H, -SO₃⁻, -OSO₃H, -OSO₃⁻, -H₂PO₃, -HPO₃⁻, -PO₃²⁻, -H₂PO₂, =HPO₂, -HPO₂⁻, =PO₂⁻, =POH, and =PO⁻ groups.

Mention may be made, as examples of anionic surfactants that may be used in the composition according to the invention, of alkyl sulfates, alkyl ether sulfates, alkylamido ether sulfates, alkylaryl polyether sulfates, monoglyceride sulfates, alkylsulfonates, alkylamidesulfonates, alkylarylsulfonates, α-olefin sulfonates, paraffin sulfonates, alkyl sulfosuccinates, alkyl ether sulfosuccinates, alkylamide sulfosuccinates, alkyl sulfoacetates, acylsarcosinates, acylglutamates, alkyl sulfosuccinamates, acylisethionates and N-acyltaurates, polyglycoside polycarboxylic acid and alkyl monoester salts, acyl lactylates, salts of D-galactoside uronic acids, salts of alkyl ether carboxylic acids, salts of alkylaryl ether carboxylic acids, salts of alkylamido ether carboxylic acids; and the corresponding non-salified forms of all these compounds; the alkyl and acyl groups of all these compounds comprising from 6 to 24 carbon atoms and the aryl group denoting a phenyl group.

These compounds may be oxyethylenated and then preferably comprise from 1 to 50 ethylene oxide units.

The salts of C₆-C₂₄ alkyl monoesters of polyglycoside-polycarboxylic acids can be selected from C₆-C₂₄ alkyl polyglycoside-citrates, C₆-C₂₄ alkyl polyglycoside-tartrates and C₆-C₂₄ alkyl polyglycoside-sulfosuccinates.

It is also possible to use monoesters of C₆₋₂₄ alkyl and of polyglycoside-polycarboxylic acids, such as alkyl glucoside citrates, alkyl polyglycoside tartrates and alkyl polyglycoside sulfosuccinates, alkyl sulfosuccinamates, acylisethionates and N-acyltaurates, the alkyl or acyl group of all these compounds comprising from 12 to 20 carbon atoms.

Another group of anionic surfactants that may be used in the composition of the present invention is that of acyl lactylates, the acyl group of which comprises from 8 to 20 carbon atoms.

In addition mention may also be made of alkyl-D-galactoside-uronic acids and their salts, and also of polyoxyalkylenated (C6-24)alkyl ether carboxylic acids, polyoxyalkylenated (C6-24)alkyl(C6-24)aryl ether carboxylic acids, polyoxyalkylenated (C6-24)alkylamido ether carboxylic acids and salts thereof, especially those containing from 2 to 50 ethylene oxide units, and mixtures thereof.

When the anionic surfactant(s) are in salt form, they may be chosen from alkali metal salts such as the sodium or potassium salt and preferably the sodium salt, ammonium salts, amine salts and in particular amino alcohol salts or alkaline-earth metal salts such as the magnesium salts.

Examples of amino alcohol salts that may especially be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.

Alkali metal or alkaline-earth metal salts, and in particular sodium or magnesium salts, are preferably used.

Among the anionic surfactants mentioned, use is preferably made of (C₆-C₂₄)alkyl sulfates, (C₆-C₂₄)alkyl ether sulfates comprising from 2 to 50 ethylene oxide units, especially in the form of alkali metal, ammonium, amino alcohol and alkaline-earth metal salts, or a mixture of these compounds.

In particular, it is preferred to use (C₁₂-C₂₀)alkyl sulfates, (C₁₂-C₂₀)alkyl ether sulfates comprising from 2 to 20 ethylene oxide units, especially in the form of alkali metal, ammonium, amino alcohol and alkaline-earth metal salts, or a mixture of these compounds. Better still, it is preferred to use sodium lauryl ether sulfate containing 2.2 mol of ethylene oxide.

The anionic surfactant(s) are present in the composition according to the invention in proportions of at least 1% by weight, preferably ranging from 3% to 50% by weight and more preferentially from 5% to 30% by weight relative to the total weight of the composition.

According to the invention, the composition comprises one or more second surfactants chosen from nonionic surfactants, and amphoteric or zwitterionic surfactants.

Examples of nonionic surfactants that may be used in the composition used according to the invention are described, for example, in the Handbook of Surfactants by M.R. Porter, published by Blackie & Son (Glasgow and London), 1991, pp. 116-178. They are especially chosen from alcohols, α-diols and (C₁-C₂₀)alkylphenols, these compounds being polyethoxylated, polypropoxylated and/or polyglycerolated, and containing at least one fatty chain comprising, for example, from 8 to 18 carbon atoms, it being possible for the number of ethylene oxide and/or propylene oxide groups to especially range from 2 to 50, and for the number of glycerol groups to especially range from 2 to 30.

Mention may also be made of copolymers of ethylene oxide and propylene oxide, optionally oxyethylenated fatty acid esters of sorbitan, fatty acid esters of sucrose, polyoxyalkylenated fatty acid esters, optionally oxyalkylenated alkylpolyglycosides, alkylglucoside esters, derivatives of N-alkylglucamine and of N-acylmethylglucamine, aldobionamides and amine oxides.

Unless otherwise mentioned, the term "fatty" compound (for example a fatty acid) denotes a compound comprising, in its main chain, at least one saturated or unsaturated hydrocarbon-based chain, such as alkyl or alkenyl containing at least 8 carbon atoms, preferably from 8 to 30 carbon atoms, and even better still from 10 to 22 carbon atoms.

The mono- or polyglycosides that may be used in the invention are well known and may be represented more particularly by the general formula (A) below:

R₁O-(R₂O)ₜ (G)ᵥ (A)

in which:
R₁ represents a linear or branched alkyl and/or alkenyl group, comprising from about 8 to 24 carbon atoms, or an alkylphenyl group whose linear or branched alkyl group comprises from 8 to 24 carbon atoms,
R₂ represents an alkylene group comprising from about 2 to 4 carbon atoms,
G represents a sugar unit comprising from 5 to 6 carbon atoms,
t denotes a value ranging from 0 to 10 and preferably 0 to 4, preferably 0 to 4, and
v denotes a value ranging from 1 to 15.

Mono- or polyglycosides that are preferred in the present invention are (C₈-C₁₈)alkyl mono- or polyglycosides and are compounds of formula (A) in which:
R₁ more particularly denotes a saturated or unsaturated, linear or branched alkyl group comprising from 8 to 18 carbon atoms,
t denotes a value ranging from 0 to 3 and even more particularly is equal to 0,
G may denote glucose, fructose or galactose, preferably glucose.

The degree of polymerization, i.e. the value of v in formula (A), may range from 1 to 15 and preferably from 1 to 4. The average degree of polymerization is more particularly between 1 and 2 and even more preferentially from 1.1 to 1.5.

The glycoside bonds between the sugar units are of 1-6 or 1-4 type and preferably of 1-4 type.

Examples of compounds of formula (A) are especially caprylylglucoside, decylglucoside or caprylglucoside, laurylglucoside, cetearylglucoside and cocoglucoside, and mixtures thereof. These preferred compounds of formula (II) are especially represented by the products sold by the company Cognis under the names Plantaren^{®} (600 CS/U, 1200 and 2000) or Plantacare^{®} (818, 1200 and 2000). It is also possible to use the products sold by the company SEPPIC under the names Triton CG 110 or Oramix CG 110 and Triton CG 312 or Oramix® NS 10, the products sold by the company BASF under the name Lutensol GD 70 or those sold by the company Chem Y under the name AG10 LK.

It is also possible to use, for example, (C₈-C₁₆)alkyl-1,4-polyglucoside as an aqueous 53% solution, sold by the company Cognis under the reference Plantacare^{®} 818 UP.

Among the cited nonionic surfactants, optionally oxyalkylenated alkylpolyglycosides are preferably used.

Among the cited nonionic surfactants, optionally oxyalkylenated alkylpolyglycosides are preferably used.

The nonionic surfactant(s) may be present in the composition according to the invention in preferential proportions of at least 0.1% by weight, preferably ranging from 0.1% to 20% by weight and more preferentially from 1% to 15% by weight relative to the total weight of the composition.

The amphoteric or zwitterionic surfactant(s), which are preferably nonsilicone, that may be used in the present invention may especially be derivatives of optionally quaternized aliphatic secondary or tertiary amines, in which derivatives the aliphatic group is a linear or branched chain comprising from 8 to 22 carbon atoms, the said amine derivatives containing at least one anionic group, for instance a carboxylate, sulfonate, sulfate, phosphate or phosphonate group. Mention may be made in particular of (C₈-C₂₀)alkylbetaines, sulfobetaines, (C₈-C₂₀)alkylamido(C₃-C₈)alkylbetaines and (C₈-C₂₀)alkylamido(C₆-C₈)alkylsulfobetaines.

Among the optionally quaternized secondary or tertiary aliphatic amine derivatives that can be used, as defined above, mention may also be made of the compounds of respective structures (A1) and (A2) below:

RₐCONHCH₂CH₂N⁺(R_{b})(R_{c})(CH₂COO⁻) (A1)

in which:
Rₐ represents a C₁₀-C₃₀ alkyl or alkenyl group derived from an acid RₐCOOH preferably present in hydrolysed coconut oil, or a heptyl, nonyl or undecyl group;
R_{b} represents a β-hydroxyethyl group, and
R_{c} represents a carboxymethyl group;
and

R_{a'}CONHCH₂CH₂N(B)(B') (A2)

in which:
B represents the group -CH₂CH₂OX',
B' represents the group -(CH₂)_{z}Y', with z = 1 or 2,
X' represents the group -CH₂COOH, CH₂COOZ', -CH₂CH₂COOH, -CH₂CH₂COOZ', or a hydrogen atom,
Y' represents the group -COOH, -COOZ', the group -CH₂CHOHSO₃H or the group -CH₂CHOHSO₃Z',
Z' represents an ion resulting from an alkali metal or alkaline earth metal, such as sodium, an ammonium ion or an ion resulting from an organic amine,
R_{a'} represents a C₁₀-C₃₀ alkyl or alkenyl group of an acid R_{a'}COON preferably present in coconut oil or in hydrolysed linseed oil, an alkyl group, especially of C₁₇ and its iso form, or an unsaturated C₁₇ group.

These compounds are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid and cocoamphodipropionic acid.

By way of example, mention may be made of the cocoamphodiacetate sold by the company Rhodia under the trade name Miranol^{®} C2M Concentrate.

Among the amphoteric or zwitterionic surfactants mentioned above, use is preferably made of (C₈-C₂₀)alkylbetaines such as cocoylbetaine, and (C₈-C₂₀)alkylamido(C₃-C₈)alkylbetaines such as cocamidopropylbetaine, and mixtures thereof. More preferentially, the amphoteric or zwitterionic surfactant(s) are chosen from cocamidopropylbetaine and cocobetaine.

The amphoteric or zwitterionic surfactant(s) may be present in the composition according to the invention in preferential proportions of at least 0.1% by weight, preferably ranging from 0.1% to 20% by weight and more preferentially from 1% to 15% by weight relative to the total weight of the composition.

The surfactant(s) are present in the compositions according to the invention such that the weight ratio between the total amount of the anionic surfactants (a) and of the said nonionic or amphoteric or zwitterionic surfactants (b), on the one hand, and the amount of solid fatty alcohol(s) according to the invention, on the other hand, is preferably greater than or equal to 2.

In one preferred embodiment, this ratio ranges from 5 to 250 and even more preferentially from 10 to 100.

Preferably, the surfactant(s) (b) are chosen from amphoteric or zwitterionic surfactants.

The solid branched fatty alcohols are solid at room temperature (25°C) and at atmospheric pressure (1 atm) and are insoluble in water (i.e. they have a solubility in water of less than 1% by weight and preferably less than 0.5% by weight, at 25°C and 1 atm) and are soluble, under the same temperature and pressure conditions, in at least one organic solvent (for example ethanol, chloroform, benzene or liquid petroleum jelly) to at least 1 % by weight.

The solid branched fatty alcohols according to the invention preferably comprise from 26 to 60 carbon atoms and especially 26 to 50 carbon atoms. The solid fatty alcohols of the invention are non-oxyalkylenated and non-glycerolated.

The melting point of the solid fatty alcohols according to the invention preferably ranges from 28 to 55°C and more particularly from 32 to 50°C.

The solid fatty alcohols according to the invention preferably have the structure R-OH, in which R denotes a branched C₂₆-C₅₀ alkyl group. R may be substituted with one or more hydroxyl groups. Preferably, R does not contain any hydroxyl groups. More particularly, the solid fatty alcohols according to the invention have the formula RaCH₂CH₂CH(Rb)CH₂OH in which Ra denotes a C₁₂-C₂₂ alkyl group and Rb denotes a C₁₆-C₂₈ alkyl group.

Examples that may be mentioned include 2-dodecylhexadecanol, 2-tetradecyl-1-octadecanol, 2-tetradecyl-1-eicosanol, 2-hexadecyl-1-octadecanol and 2-hexadecyl-1-eicosanol, and mixtures thereof.

The solid fatty alcohols may be mixtures, which means that several species may coexist in a commercial product, especially of different chain lengths, in the form of a mixture.

The solid branched fatty alcohol(s) of the invention are generally present in an amount ranging from 0.01% to 10% by weight, preferentially from 0.03% to 5% by weight and better still from 0.05% to 3% by weight, relative to the total weight of the composition.

The composition according to the invention may also especially comprise one or more cationic polymers. The cationic polymers that may be used in accordance with the present invention may be chosen from any of those already known per se as improving the cosmetic properties of hair, namely, especially, those described in patent application EP-A-0 337 354 and in French patent applications FR-A-2 270 846, 2 383 660, 2 598 611, 2 470 596 and 2 519 863.

Even more generally, for the purposes of the present invention, the term "cationic polymer" denotes any polymer comprising cationic groups and/or groups that can be ionized into cationic groups.

The preferred cationic polymers are chosen from those that contain units comprising primary, secondary, tertiary and/or quaternary amine groups that may either form part of the main polymer chain or may be borne by a side substituent directly connected thereto.

The cationic polymers used generally have a number-average or weight-average molar mass of between 500 and 5×10⁶ approximately and preferably between 10³ and 3×10⁶ approximately.

Among the cationic polymers that may be mentioned more particularly are polymers of the polyamine, polyaminoamide and polyquaternary ammonium type. These are known products.

The polymers of polyamine, polyamidoamide and polyquaternary ammonium type, that can be used in accordance with the present invention, and that can in particular be mentioned, are those described in French patents No. 2 505 348 or 2 542 997. Among these polymers, mention may be made of:
(1) homopolymers or copolymers derived from acrylic or methacrylic esters or amides and comprising at least one of the units of the following formulae: 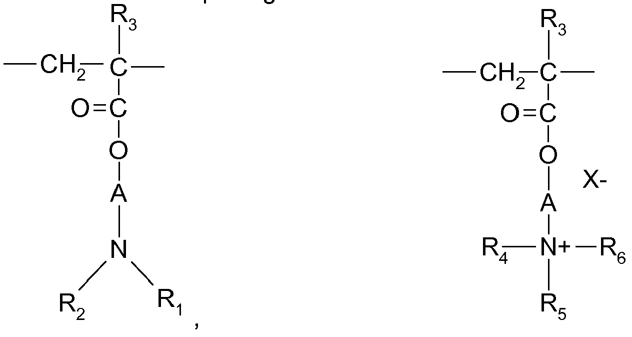 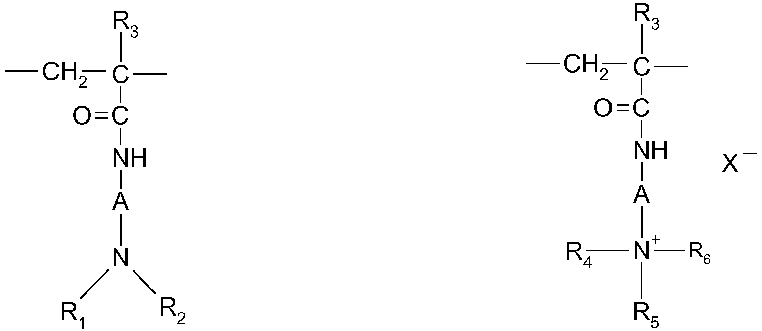 in which:
   R₃, which may be identical or different, denotes a hydrogen atom or a CH₃ radical;
   A, which may be identical or different, represent a linear or branched alkyl group of 1 to 6 carbon atoms, preferably 2 or 3 carbon atoms, or a hydroxyalkyl group of 1 to 4 carbon atoms;
   R₄, R₅ and R₆, which may be identical or different, represent an alkyl group containing from 1 to 18 carbon atoms or a benzyl radical and preferably an alkyl group containing from 1 to 6 carbon atoms;
   R₁ and R₂, which may be identical or different, represent hydrogen or an alkyl group containing from 1 to 6 carbon atoms, and preferably methyl or ethyl;
   X denotes an anion derived from a mineral or organic acid, such as a methosulfate anion or a halide such as chloride or bromide.

The copolymers of family (1) can also contain one or more units derived from comonomers that may be selected from the family of acrylamides, methacrylamides, diacetone acrylamides, acrylamides and methacrylamides substituted on the nitrogen with lower (C₁-C₄) alkyls, acrylic or methacrylic acids or esters thereof, vinyllactams such as vinylpyrrolidone or vinylcaprolactam, and vinyl esters.

Thus, among these copolymers of the family (1), mention may be made of:
- copolymers of acrylamide and of dimethylaminoethyl methacrylate quaternized with dimethyl sulfate or with a dimethyl halide, such as the product sold under the name Hercofloc by the company Hercules,
- copolymers of acrylamide and of methacryloyloxyethyltrimethylammonium chloride, described, for example, in Patent Application EP-A-080 976 and sold under the name Bina Quat P 100 by the company Ciba Geigy,
- copolymers of acrylamide and of methacryloyloxyethyltrimethylammonium methosulfate, such as the product sold under the name Reten by the company Hercules,
- quaternized or non-quaternized vinylpyrrolidone/dialkylaminoalkyl acrylate or methacrylate copolymers, such as the products sold under the name Gafquat by the company ISP, such as, for example, Gafquat 734 or Gafquat 755, or alternatively the products known as Copolymer 845, 958 and 937. These polymers are described in detail in French patents 2 077 143 and 2 393 573,
- dimethylaminoethyl methacrylate/vinylcaprolactam/vinylpyrrolidone terpolymers, such as the product sold under the name Gaffix VC 713 by the company ISP,
- vinylpyrrolidone/methacrylamidopropyldimethylamine copolymers sold in particular under the name Styleze CC 10 by ISP,
- quaternized vinylpyrrolidone/dimethylaminopropylmethacrylamide copolymers such as the product sold under the name Gafquat HS 100 by the company ISP, and
- preferably crosslinked polymers of methacryloyloxy(C₁-C₄)alkyl tri(C₁-C₄)alkylammonium salts, such as the polymers obtained by homopolymerization of dimethylaminoethyl methacrylate quaternized with methyl chloride, or by copolymerization of acrylamide with dimethylaminoethyl methacrylate quaternized with methyl chloride, the homo- or copolymerization being followed by crosslinking with an olefinically unsaturated compound, more particularly methylenebisacrylamide. A crosslinked acrylamide/methacryloyloxyethyltrimethylammonium chloride copolymer (for example 20/80 by weight) in particular in the form of a dispersion containing 50% by weight of the said copolymer in mineral oil can be used more particularly. This dispersion is sold under the name Salcare^{®} SC 92 by the company Ciba. A crosslinked methacryloyloxyethyltrimethylammonium chloride homopolymer especially comprising about 50% by weight of the homopolymer in mineral oil or in a liquid ester may also be used. These dispersions are sold under the names Salcare^{®} SC 95 and Salcare^{®} SC 96 by the company Ciba.

(2) Cationic polysaccharides, especially cationic celluloses and galactomannan gums. Among the cationic polysaccharides, mention may be made more particularly of cellulose ether derivatives comprising quaternary ammonium groups, cationic cellulose copolymers or cellulose derivatives grafted with a water-soluble quaternary ammonium monomer and cationic galactomannan gums.

The cellulose ether derivatives containing quaternary ammonium groups, described in French patent 1 492 597, and in particular polymers sold under the names "JR" (JR 400, JR 125 and JR 30M) or "LR" (LR 400 or LR 30M) by the company Amerchol. These polymers are also defined in the CTFA dictionary as quaternary ammoniums of hydroxyethyl cellulose that have reacted with an epoxide substituted with a trimethylammonium group.

Cationic cellulose copolymers or cellulose derivatives grafted with a water-soluble monomer of quaternary ammonium are described especially in US patent 4 131 576, such as hydroxyalkyl celluloses, for instance hydroxymethyl-, hydroxyethyl- or hydroxypropylcelluloses grafted, in particular, with a methacryloylethyltrimethylammonium, methacrylamidopropyltrimethylammonium or dimethyldiallylammonium salt.

The commercial products corresponding to this definition are more particularly the products sold under the names Celquat L 200 and Celquat H 100 by the company National Starch.

The cationic galactomannan gums are described more particularly in US patents 3 589 578 and 4 031 307, in particular guar gums comprising cationic trialkylammonium groups. Guar gums modified with a salt (e.g. chloride) of 2,3-epoxypropyltrimethylammonium are used, for example.

Such products are sold in particular under the trade names Jaguar C13 S, Jaguar C 15, Jaguar C 17 or Jaguar C162 by the company Rhodia.
(3) Polymers formed from piperazinyl units and divalent alkylene or hydroxyalkylene radicals containing straight or branched chains, optionally interrupted with oxygen, sulfur or nitrogen atoms or with aromatic or heterocyclic rings, and also the oxidation and/or quaternization products of these polymers. Such polymers are especially described in French Patents 2 162 025 and 2 280 361.
(4) Water-soluble polyaminoamides prepared in particular by polycondensation of an acidic compound with a polyamine; these polyaminoamides can be crosslinked with an epihalohydrin, a diepoxide, a dianhydride, an unsaturated dianhydride, a bisunsaturated derivative, a bis-halohydrin, a bisazetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyaminoamide; these polyaminoamides can be alkylated or, if they comprise one or more tertiary amine functions, they can be quaternized. Such polymers are especially described in French Patents 2 252 840 and 2 368 508.
(5) Polyaminoamide derivatives resulting from the condensation of polyalkylene polyamines with polycarboxylic acids followed by alkylation with difunctional agents. Mention may be made, for example, of adipic acid/dialkylaminohydroxyalkyldialkylenetriamine polymers in which the alkyl radical comprises from 1 to 4 carbon atoms and preferably denotes methyl, ethyl or propyl. Such polymers are especially described in French patent 1 583 363.

Among these derivatives, mention may be made more particularly of the adipic acid/dimethylaminohydroxypropyl/diethylenetriamine polymers sold under the name Cartaretine F, F4 or F8" by the company Sandoz.
(6) The polymers obtained by reaction of a polyalkylene polyamine containing two primary amine groups and at least one secondary amine group with a dicarboxylic acid selected from diglycolic acid and saturated aliphatic dicarboxylic acids having from 3 to 8 carbon atoms. The molar ratio between the polyalkylene polyamine and the dicarboxylic acid is between 0.8:1 and 1.4:1; the polyamino amide resulting therefrom is reacted with epichlorohydrin in a mole ratio of epichlorohydrin relative to the secondary amine group of the polyamino amide of between 0.5:1 and 1.8:1. Such polymers are described in particular in US patents 3 227 615 and 2 961 347.

Polymers of this type are sold in particular under the name Hercosett 57 by the company Hercules Inc. or alternatively under the name PD 170 or Delsette 101 by the company Hercules in the case of the adipic acid/epoxypropyl/diethylenetriamine copolymer.
(7) Cyclopolymers of alkyldiallylamine or of dialkyldiallylammonium, such as the homopolymers or copolymers containing, as main constituent of the chain, units corresponding to formula (I) or (II): 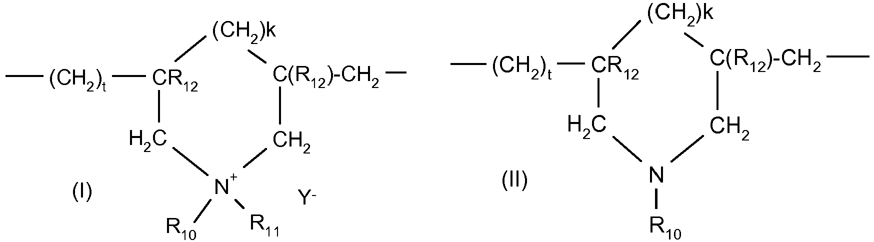 in which formulae k and t are equal to 0 or 1, the sum k + t being equal to 1; R₁₂ denotes a hydrogen atom or a methyl group; R₁₀ and R₁₁, independently of one another, denote an alkyl group having from 1 to 6 carbon atoms, a hydroxyalkyl group in which the alkyl group has preferably 1 to 5 carbon atoms, a lower (C₁-C₄) amidoalkyl group, or else R₁₀ and R₁₁ may, together with the nitrogen atom to which they are attached, denote heterocyclic groups, such as piperidyl or morpholinyl; Y⁻ is an anion such as bromide, chloride, acetate, borate, citrate, tartrate, bisulfate, bisulfite, sulfate or phosphate. These polymers are in particular described in French patent 2 080 759 and in its Certificate of Addition 2 190 406.

R₁₀ and R₁₁, independently of one another, denote preferably an alkyl group having from 1 to 4 carbon atoms.

Among the polymers defined above, mention may be made more particularly of the dimethyldiallylammonium salt (for example chloride) homopolymer sold under the name Merquat 100 by the company Nalco (and homologues thereof of low weight-average molar masses) and the copolymers of diallyldimethylammonium salts (for example chloride) and of acrylamide, sold especially under the name Merquat 550.
(8) The quaternary diammonium polymers comprising repeating units corresponding to the formula: 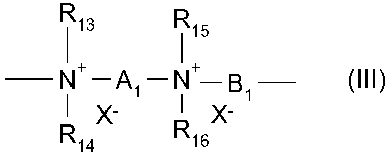 in which formula (III):
   R₁₃, R₁₄, R₁₅ and R₁₆, which may be identical or different, represent aliphatic, alicyclic or arylaliphatic radicals comprising from 1 to 20 carbon atoms or lower hydroxyalkylaliphatic radicals, or alternatively R₁₃, R₁₄, R₁₅ and R₁₆, together or separately, constitute, with the nitrogen atoms to which they are attached, heterocycles optionally comprising a second heteroatom other than nitrogen, or alternatively R₁₃, R₁₄, R₁₅ and R₁₆ represent a linear or branched C₁-C₆ alkyl radical substituted with a nitrile, ester, acyl or amide group or a group COOR₁₇D or CONHR₁₇D where R₁₇ is an alkylene and D is a quaternary ammonium group,
   A₁ and B₁ represent polymethylene groups comprising from 2 to 20 carbon atoms, which may be linear or branched, saturated or unsaturated, and which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and
   X- denotes an anion derived from an inorganic or organic acid;
   A₁, R₁₃ and R₁₅ may form, with the two nitrogen atoms to which they are attached, a piperazine ring; moreover, if A₁ denotes a saturated or unsaturated, linear or branched alkylene or hydroxyalkylene radical, B₁ may also denote a group (CH₂)ₙ-CO-D-OC-(CH₂)ₙ-
   in which D denotes:
   a) a glycol residue of formula: -O-Z-O-, where Z denotes a linear or branched hydrocarbon-based radical or a group corresponding to one of the following formulae:

      -(CH₂-CH₂-O)x-CH₂-CH₂-

      -[CH₂-CH(CH₃)-O]_{y}-CH₂-CH(CH₃)-

      in which x and y denote an integer from 1 to 4, representing a defined and unique degree of polymerization or any number from 1 to 4 representing an average degree of polymerization;
   b) a bis-secondary diamine residue such as a piperazine derivative;
   c) a bis-primary diamine residue of formula: -NH-Y-NH-, where Y denotes a linear or branched hydrocarbon-based radical, or alternatively the divalent radical

      -CH₂-CH₂-S-S-CH₂-CH₂-;
   d) a ureylene group of formula: -NH-CO-NH-;

Preferably, X- is an anion such as chloride or bromide.

These polymers generally have a number-average molecular mass generally of between 1000 and 100 000.

Polymers of this type are described in particular in French patents 2 320 330, 2 270 846, 2 316 271, 2 336 434 and 2 413 907 and US patents 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 and 4 027 020.

Use may be made more particularly of polymers that are composed of repeating units corresponding to the formula: 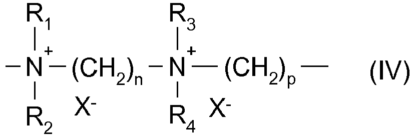 in which R₁, R₂, R₃ and R₄, which may be identical or different, denote an alkyl or hydroxyalkyl radical containing from 1 to 4 carbon atoms approximately, n and p are integers ranging from 2 to 20 approximately, and X- is an anion derived from a mineral or organic acid.

One particularly preferred compound of formula (IV) is that for which R₁, R₂, R₃ and R₄ represent a methyl group and n = 3, p = 6 and X = Cl, which is called Hexadimethrine chloride according to INCI nomenclature (CTFA).
(9) Polyquaternary ammonium polymers comprising units of formula (V): 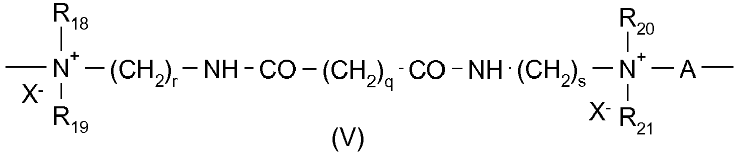 in which formula:
   R₁₈, R₁₉, R₂₀ and R₂₁, which may be identical or different, represent a hydrogen atom or a methyl, ethyl, propyl, β-hydroxyethyl, β-hydroxypropyl or - CH₂CH₂(OCH₂CH₂)ₚOH group,
   where p is equal to 0 or to an integer between 1 and 6, with the proviso that R₁₈, R₁₉, R₂₀ and R₂₁ do not simultaneously represent a hydrogen atom,
   r and s, which may be identical or different, are integers between 1 and 6,
   q is equal to 0 or to an integer between 1 and 34,
   X- denotes an anion such as a halide,
   A denotes a dihalide radical or preferably represents -CH₂-CH₂-O-CH₂-CH₂-

Such compounds are described especially in Patent Application EP-A-122 324.

Among these, mention may be made, for example, of the products Mirapol® A 15, Mirapol® AD1, Mirapol® AZ1 and Mirapol® 175, sold by the company Miranol.
(10) Quaternary polymers of vinylpyrrolidone and of vinylimidazole, for instance the products sold under the names Luviquat® FC 905, FC 550 and FC 370 by the company BASF.
(11) Polyamines such as Polyquart® H sold by Cognis, referred to under the name Polyethylene glycol (15) tallow polyamine in the CTFA dictionary.

Other cationic polymers that may be used in the context of the invention are cationic proteins or cationic protein hydrolysates, polyalkyleneimines, in particular polyethyleneimines, polymers comprising vinylpyridine or vinylpyridinium units, condensates of polyamines and of epichlorohydrin, quaternary polyureylenes and chitin derivatives.

Among the cationic polymers mentioned above, which are suitable in the invention, the ones that may preferably be used are quaternary cellulose ether derivatives such as the products sold under the name "JR 400" by the company Amerchol, cationic cyclopolymers, in particular dimethyldiallylammonium salt (for example chloride) homopolymers or copolymers, products sold under the names Merquat 100, Merquat 550 and Merquat S by the company Nalco, and homologues thereof of low weight-average molecular weights, quaternary polymers of vinylpyrrolidone and of vinylimidazole, optionally crosslinked homopolymers or copolymers of methacryloyloxy(C₁-C₄)alkyltri(C₁-C₄)alkylammonium salts, and mixtures thereof.

The cationic polymer(s) are generally present in concentrations ranging from 0.01% to 20% by weight, preferably from 0.05% to 10% by weight and more particularly from 0.1% to 5% by weight, relative to the total weight of the composition.

The composition according to the invention may also especially comprise at least one silicone. This silicone may be linear, branched or cyclic, volatile or nonvolatile, and organomodified or non-organomodified.

Preferably, the composition of the invention comprises water or a mixture of water and of at least one organic solvent chosen from C₁-C₄ lower alcohols, such as ethanol, isopropanol, tert-butanol or n-butanol; polyols such as glycerol, propylene glycol and polyethylene glycols.

Preferably, the composition comprises from 70% to 95% by weight of water relative to the total weight of the composition.

The pH of the compositions according to the invention is generally between 2 and 11, preferably between 3 and 10 and better still between 4 and 8.

The composition according to the invention may also comprise additives chosen from anionic polymers, nonionic polymers, amphoteric polymers, associative or non-associative polymeric thickeners, non-polymeric thickeners, cationic surfactants, nacreous agents, opacifiers, dyes or pigments, fragrances, mineral, plant or synthetic oils, waxes including ceramides, vitamins, UV-screening agents, free-radical scavengers, antidandruff agents, hair-loss counteractants, hair restorers, preserving agents, pH stabilizers and solvents, and mixtures thereof. A person skilled in the art will take care to select the optional additives and the amount thereof such that they do not harm the properties of the compositions of the present invention.

These additives are generally present in the composition according to the invention in an amount ranging from 0 to 20% by weight relative to the total weight of the composition.

The compositions may be packaged in various forms, especially in bottles, in pump bottles or in aerosol containers so as to apply the composition in vaporized form or in the form of a mousse. The compositions may also impregnate applicators, especially gloves or wipes.

The present invention also relates to a process for washing keratin materials, which consists in applying an effective amount of a composition as defined above to the said keratin materials, and in rinsing, for example with water, after an optional leave-on time.

The present invention also relates to a process for cleansing keratin materials, which consists in applying an effective amount of a composition as defined above to the said keratin materials, and in optionally rinsing, for example with water, after an optional leave-on time.

The examples that follow are given as illustrations of the present invention. Unless otherwise mentioned, all the amounts indicated are expressed as weight percentages.

### Example 1

The following shampoo composition was prepared:

| Composition | Invention | |
|---|---|---|
| Sodium lauryl ether sulfate (70/30 C12/C14) containing 2.2 mol of ethylene oxide (Texapon AOS 225 UP from Cognis) | 5.25 | g AM |
| Sodium lauryl sulfate (70/30 C12/C14) (Texapon LS 35 from Cognis) | 4.06 | g AM |
| Disodium cocoamphodiacetate (Miranol C2M Conc. NP from Rhodia) | 1.52 | g AM |
| 2-Tetradecyloctadecanol (Isofol 32 from Sasol) | 0.05 | g |
| Oxyethylenated (60 EO) cetylstearyl alcohol (C16/C18) ether of myristyl glycol (Elfacos GT 282 S from Akzo Nobel) | 2.5 | g |
| Polyquaternium-10 (Polymer JR400 LT from Amerchol) | 0.1 | g |
| Sodium chloride | 0.5 | g |
| Preserving agents, fragrance | qs | |
| pH agent qs | pH | 5.3 |
| Demineralized water qs | 100 | g |

| | | |
|---|---|---|
| *: as Active material (AM) | | |

Hair treated with the composition of Example 1 is smooth and easy to disentangle.

### Example 2

The following shampoo composition was prepared:

| Composition | Example 2 | |
|---|---|---|
| Sodium lauryl ether sulfate (70/30 C12/C14) containing 2.2 mol of ethylene oxide (Texapon AOS 225 UP from Cognis) | 5.25 | g AM |
| Sodium lauryl sulfate (70/30 C12/C14) (Texapon LS 35 from Cognis) | 4.06 | g AM |
| Disodium cocoamphodiacetate (Miranol C2M Conc. NP from Rhodia) | 1.52 | g AM |
| Hydroxypropyl guar trimethylammonium chloride (Jaguar C162 from Rhodia) | 0.15 | g |
| 2-Tetradecyloctadecanol (Isofol 32 from Sasol) | 0.2 | g |
| Oxyethylenated (60 EO) cetylstearyl alcohol (C16/C18) ether of myristyl glycol (Elfacos GT 282 S from Akzo Nobel) | 3 | g |
| Preserving agents, fragrance | qs | |
| pH agent qs | pH | 5.3 |
| Demineralized water qs | 100 | g |

Hair treated with the composition of Example 2 is smooth and easy to disentangle.

## Claims

1. Composition, especially for washing or cleansing keratin materials, **characterized in that** it comprises:
a) one or more anionic surfactants,
b) one or more surfactants chosen from nonionic surfactants, and amphoteric or zwitterionic surfactants, and
c) one or more solid branched fatty alcohols containing more than 25 carbon atoms.

2. Composition according to Claim 1, **characterized in that** the anionic surfactant(s) are chosen from alkyl sulfates, alkyl ether sulfates, alkylamido ether sulfates, alkylaryl polyether sulfates, monoglyceride sulfates, alkylsulfonates, alkylamidesulfonates, alkylarylsulfonates, α-olefin sulfonates, paraffin sulfonates, alkylsulfosuccinates, alkyl ether sulfosuccinates, alkylamide sulfosuccinates, alkylsulfoacetates, acylsarcosinates, acylglutamates, alkylsulfosuccinamates, acylisethionates and N-acyltaurates, salts of alkyl monoesters and of polyglycoside polycarboxylic acids, acyllactylates, D-galactoside uronic acid salts, alkyl ether carboxylic acid salts, alkylaryl ether carboxylic acid salts, alkylamido ether carboxylic acid salts, preferably from (C₁₂-C₂₀)alkyl sulfates, (C₁₂-C₂₀)alkyl ether sulfates comprising from 2 to 20 ethylene oxide units, and more preferentially from sodium lauryl sulfate and sodium lauryl ether sulfate containing 2.2 mol of ethylene oxide.

3. Composition according to either of the preceding claims, **characterized in that** the anionic surfactant(s) are present in proportions of at least 1% by weight, preferably from 3% to 50% by weight and more preferentially from 5% to 30% by weight relative to the total weight of the composition.

4. Composition according to any one of the preceding claims, **characterized in that** the nonionic surfactant(s) are chosen from:
- alcohols, α-diols and (C₁-C₂₀)alkylphenols, these compounds being polyethoxylated, polypropoxylated and/or polyglycerolated, and containing at least one fatty chain comprising, for example, from 8 to 18 carbon atoms, the number of ethylene oxide and/or propylene oxide groups possibly ranging especially from 2 to 50, and the number of glycerol groups possibly ranging especially from 2 to 30,
- copolymers of ethylene oxide and propylene oxide, optionally oxyethylenated fatty acid esters of sorbitan, fatty acid esters of sucrose, polyoxyalkylenated fatty acid esters, optionally oxyalkylenated alkylpolyglycosides, alkylglucoside esters, derivatives of N-alkylglucamine and of N-acylmethylglucamine, aldobionamides and amine oxides,
and preferably from optionally oxyalkylenated alkylpolyglycosides.

5. Composition according to any one of the preceding claims, **characterized in that** the amphoteric or zwitterionic surfactant(s) are chosen from (C₈-C₂₀)alkylbetaines, sulfobetaines, (C₈-C₂₀)alkylamido(C₃-C₈)alkylbetaines and (C₈-C₂₀)alkylamido(C₆-C₈)alkylsulfobetaines, preferably from cocoylbetaine and cocamidopropylbetaine.

6. Composition according to any one of the preceding claims, **characterized in that** the surfactant(s) (b) are chosen from amphoteric or zwitterionic surfactants.

7. Composition according to any one of the preceding claims, **characterized in that** the nonionic, amphoteric or zwitterionic surfactant(s) are present in proportions of at least 0.1% by weight, preferably ranging from 0.1% to 20% by weight and more preferentially from 1% to 15% by weight relative to the total weight of the composition.

8. Composition according to any one of the preceding claims, **characterized in that** the solid branched fatty alcohols containing more than 25 carbon atoms comprise from 26 to 60 carbon atoms and especially from 26 to 50 carbon atoms.

9. Composition according to any one of the preceding claims, **characterized in that** the solid branched fatty alcohols containing more than 25 carbon atoms are chosen from 2-dodecylhexadecanol, 2-tetradecyl-1-octadecanol, 2-tetradecyl-1-eicosanol, 2-hexadecyl-1-octadecanol and 2-hexadecyl-1-eicosanol, and mixtures thereof.

10. Composition according to any one of the preceding claims, **characterized in that** the solid branched fatty alcohol(s) containing more than 25 carbon atoms are present in an amount ranging from 0.01% to 10% by weight, preferentially from 0.03% to 5% by weight and better still from 0.05% to 3% by weight relative to the total weight of the composition.

11. Composition according to any one of the preceding claims, **characterized in that** the weight ratio between the total amount of anionic surfactants (a) and of the said nonionic or amphoteric or zwitterionic surfactant(s) (b), on the one hand, and the amount of solid branched fatty alcohol(s) containing more than 25 carbon atoms, on the other hand, is greater than or equal to 2, preferably ranging from 5 to 250 and even more preferentially from 10 to 100.

12. Composition according to any one of the preceding claims, **characterized in that** it comprises other ingredients chosen from silicones, anionic polymers, cationic polymers, nonionic polymers, amphoteric polymers, associative or non-associative polymeric thickeners, non-polymeric thickeners, cationic surfactants, nacreous agents, opacifiers, dyes or pigments, fragrances, mineral, plant or synthetic oils, waxes including ceramides, vitamins, UV-screening agents, free-radical scavengers, antidandruff agents, hair-loss counteractants, hair restorers, preserving agents, pH stabilizers and solvents, and mixtures thereof.

13. Use of the composition according to any one of the preceding claims, for washing, cleansing and conditioning keratin materials and in particular keratin fibres.

14. Process for washing, cleansing and conditioning keratin materials and in particular keratin fibres, comprising the application of an effective amount of a composition according to any one of Claims 1 to 12 to the said keratin materials, and rinsing after an optional leave-on time.

## Patentansprüche

1. Zusammensetzung, insbesondere zum Waschen oder Reinigen von Keratinmaterialien, **dadurch gekennzeichnet, dass** sie Folgendes umfasst:
a) ein oder mehrere anionische Tenside,
b) ein oder mehrere Tenside, die aus nichtionischen Tensiden und amphoteren oder zwitterionischen Tensiden ausgewählt sind, und
c) ein oder mehrere feste verzweigte Fettalkohole mit mehr als 25 Kohlenstoffatomen.

2. Zusammensetzung nach Anspruch 1, **dadurch gekennzeichnet, dass** das anionische Tensid bzw. die anionischen Tenside aus Alkylsulfaten, Alkylether-sulfaten, Alkylamidoethersulfaten, Alkylarylpolyethersulfaten, Monoglyceridsulfaten, Alkylsulfonaten, Alkylamidsulfonaten, Alkylarylsulfonaten, α-Olefinsulfonaten, Paraffinsulfonaten, Alkylsulfosuccinaten, Alkylethersulfosuccinaten, Alkylamidsulfosuccinaten, Alkylsulfo-acetaten, Acylsarcosinaten, Acylglutamaten, Alkylsulfosuccinamaten, Acylisethionaten und N-Acyltauraten, Salzen von Alkylmonoestern und von Polyglycosidpolycarbonsäuren, Acyllactylaten, D-Galactosiduronsäuresalzen, Alkylethercarbonsäuresalzen, Alkylarylethercarbonsäuresalzen, Alkylamidoethercarbonsäuresalzen, vorzugsweise aus (C₁₂-C₂₀)Alkylsulfaten, (C₁₂-C₂₀)Alkylethersulfaten mit 2 bis 20 Ethylenoxid-Einheiten und weiter bevorzugt aus Natriumlaurylsulfat und Natriumlaurylethersulfat mit 2,2 mol Ethylenoxid, ausgewählt ist bzw. sind.

3. Zusammensetzung nach einem der vorhergehenden Ansprüche, **dadurch gekennzeichnet, dass** das anionische Tensid bzw. die anionischen Tenside in Anteilen von mindestens 1 Gew.-%, vorzugsweise 3 bis 50 Gew.-% und weiter bevorzugt 5 bis 30 Gew.-%, bezogen auf das Gesamtgewicht der Zusammensetzung, vorliegt bzw. vorliegen.

4. Zusammensetzung nach einem der vorhergehenden Ansprüche, **dadurch gekennzeichnet, dass** das nichtionische Tensid bzw. die nichtionischen Tenside aus:
- Alkoholen, α-Diolen und (C₁-C₂₀)Alkylphenolen, wobei diese Verbindungen polyethoxyliert, polypropoxyliert und/oder polyglyceriniert sind und mindestens eine Fettsäurekette mit beispielsweise 8 bis 18 Kohlenstoffatomen enthalten, wobei die Zahl der Ethylenoxid-und/oder Propylenoxidgruppen gegebenenfalls insbesondere im Bereich von 2 bis 50 liegt und die Zahl der Glyceringruppen gegebenenfalls insbesondere im Bereich von 2 bis 30 liegt,
- Copolymeren von Ethylenoxid um Propylenoxid, gegebenenfalls oxyethylenierten Fettsäureestern von Sorbitan, Fettsäureestern von Saccharose, polyoxyalkylenierten Fettsäureestern, gegebenenfalls oxyalkylenierten Alkylpolyglycosiden, Alkylglucosidestern, Derivaten von N-Alkylglucamin und von N-Acylmethylglucamin, Aldobionamiden und Aminoxiden
und vorzugsweise aus gegebenenfalls oxyalkylenierten Alkylpolyglycosiden
ausgewählt ist bzw. sind.

5. Zusammensetzung nach einem der vorhergehenden Ansprüche, **dadurch gekennzeichnet, dass** das amphotere oder zwitterionische Tensid bzw. die amphoteren oder zwitterionischen Tenside aus C₈-C₂₀)Alkylbetainen, Sulfobetainen, (C₈-C₂₀)Alkylamido(C₃-C₈)alkylbetainen und (C₈-C₂₀)Alkylamido(C₆-C₈)alkylsulfobetainen, vorzugsweise aus Cocoylbetain und Cocamidopropylbetain ausgewählt ist bzw. sind.

6. Zusammensetzung nach einem der vorhergehenden Ansprüche, **dadurch gekennzeichnet, dass** das Tensid bzw. die Tenside (b) aus amphoteren oder zwitterionischen Tensiden ausgewählt ist bzw. sind.

7. Zusammensetzung nach einem der vorhergehenden Ansprüche, **dadurch gekennzeichnet, dass** das nichtionische, amphotere oder zwitterionische Tensid bzw. die nichtionischen, amphoteren oder zwitterionischen Tenside in Anteilen von mindestens 0,1 Gew.-%, vorzugsweise im Bereich von 0,1 bis 20 Gew.-% und weiter bevorzugt von 1 bis 50 Gew.-%, bezogen auf das Gesamtgewicht der Zusammensetzung, vorliegt bzw. vorliegen.

8. Zusammensetzung nach einem der vorhergehenden Ansprüche, **dadurch gekennzeichnet, dass** die festen verzweigten Fettalkohole mit mehr als 25 Kohlenstoffatomen 26 bis 60 Kohlenstoffatome und insbesondere 26 bis 50 Kohlenstoffatome umfassen.

9. Zusammensetzung nach einem der vorhergehenden Ansprüche, **dadurch gekennzeichnet, dass** die festen verzweigten Fettalkohole mit mehr als 25 Kohlenstoffatomen aus 2-Dodecylhexadecanol, 2-Tetradecyl-1-octadecanol, 2-Tetradecyl-1-eicosanol, 2-Hexadecyl-1-octadecanol und 2-Hexadecyl-1-eicosanol und Mischungen davon ausgewählt sind.

10. Zusammensetzung nach einem der vorhergehenden Ansprüche, **dadurch gekennzeichnet, dass** der feste verzweigte Fettalkohol bzw. die festen verzweigten Fettalkohole mit mehr als 25 Kohlenstoffatomen in einer Menge im Bereich von 0,01 bis 10 Gew.-%, vorzugsweisen 0,03 bis 5 Gew.-% und noch besser 0,05 bis 3 Gew.-%, bezogen auf das Gesamtgewicht der Zusammensetzung, vorliegt bzw. vorliegen.

11. Zusammensetzung nach einem der vorhergehenden Ansprüche, **dadurch gekennzeichnet, dass** das Gewichtsverhältnis zwischen der Gesamtmenge von anionischem Tensid bzw. anionischen Tensiden (a) und dem nichtionischen oder amphoteren oder zwitterionischen Tensid bzw. dem nichtionischen oder amphoteren oder zwitterionischen Tensiden (b) einerseits und der Menge von festem verzweigtem Fettalkohol bzw. festen verzweigten Fettalkoholen mit mehr als 25 Kohlenstoffatomen andererseits größer gleich 2 ist und vorzugsweise im Bereich von 5 bis 250 und noch weiter bevorzugt von 10 bis 100 liegt.

12. Zusammensetzung nach einem der vorhergehenden Ansprüche, **dadurch gekennzeichnet, dass** sie andere Bestandteile umfasst, die aus Silikonen, anionischen Polymeren, kationischen Polymeren, nichtionischen Polymeren, amphoteren Polymeren, assoziativen oder nicht assoziativen polymeren Verdickern, nichtpolymeren Verdickern, kationischen Tensiden, Perlglanzmitteln, Trübungsmitteln, Farbstoffen oder Pigmenten, Duftstoffen, mineralischen, pflanzlichen oder synthetischen Ölen, Wachsen einschließlich Ceramiden, Vitaminen, UV-Schutzmitteln, Radikalfängern, Antischuppenmitteln, Mitteln zur Bekämpfung von Haarverlust, Haarwuchsmitteln, Konservierungsstoffen, pH-Stabilisatoren und Lösungsmitteln und Mischungen davon ausgewählt sind.

13. Verwendung der Zusammensetzung nach einem der vorhergehenden Ansprüche zum Waschen, Reinigen und Konditionieren von Keratinmaterialien und insbesondere Keratinfasern.

14. Verfahren zum Waschen, Reinigen und Konditionieren von Keratinmaterialien und insbesondere Keratinfasern, bei dem man auf die Keratinmaterialien eine wirksame Menge einer Zusammensetzung nach einem der Ansprüche 1 bis 12 aufbringt und nach einer fakultativen Einwirkungszeit spült.

## Revendications

1. Composition, notamment destinée au lavage ou au nettoyage des matériaux kératiniques, **caractérisée en ce qu'**elle comprend :
a) un ou plusieurs agents tensioactifs anioniques,
b) un ou plusieurs agents tensioactifs choisis parmi les agents tensioactifs non ioniques, et les agents tensioactifs amphotères ou zwitterioniques, et
c) un ou plusieurs alcools gras ramifiés solides contenant plus de 25 atomes de carbone.

2. Composition selon la revendication 1, **caractérisée en ce que** le ou les agents tensioactifs anioniques sont choisis parmi les alkylsulfates, les alkyléther sulfates, les alkylamido éther sulfates, les alkylaryl polyéther sulfates, les sulfates de monoglycéride, les alkylsulfonates, les alkylamide sulfonates, les alkylarylsulfonates, les sulfonates d'α-oléfine, les sulfonates de paraffine, les alkylsulfosuccinates, les alkyléther sulfosuccinates, les alkylamide sulfosuccinates, les alkylsulfoacétates, les acylsarcosinates, les acylglutamates, les alkylsulfosuccinamates, les acyliséthionates et les N-acyltaurates, les sels de monoesters d'alkyle et d'acides polyglycoside-polycarboxyliques, les acyllactylates, les sels d'acide D-galactosideuronique, les sels d'acide alkyléther carboxylique, les sels d'acide alkylaryléther carboxylique, les sels d'acide alkylamidoéther carboxylique, préférablement les (C₁₂-C₂₀)alkylsulfates, les (C₁₂-C₂₀) alkyléther sulfates comprenant de 2 à 20 unités d'oxyde d'éthylène, et plus préférablement le laurylsulfate de sodium et le lauryl éther sulfate de sodium contenant 2,2 mol d'oxyde d'éthylène.

3. Composition selon l'une ou l'autre parmi les revendications précédentes, **caractérisée en ce que** le ou les agents tensioactifs anioniques sont présents selon des proportions d'au moins 1% en poids, préférablement de 3% à 50% en poids et plus préférablement de 5% à 30% en poids, par rapport au poids total de la composition.

4. Composition selon l'une quelconque des revendications précédentes, **caractérisée en ce que** le ou les agents tensioactifs non ioniques sont choisis parmi :
- les alcools, α-diols et (C₁-C₂₀)alkylphénols, ces composés étant polyéthoxylés, polypropoxylés et/ou polyglycérolés, et contenant au moins un chaîne grasse comprenant par exemple de 8 à 18 atomes de carbone, le nombre de groupements oxyde d'éthylène et/ou oxyde de propylène allant éventuellement notamment de 2 à 50, et le nombre de groupements glycérol allant éventuellement notamment de 2 à 30,
- les copolymères d'oxyde d'éthylène et d'oxyde de propylène, les esters d'acides gras et de sorbitane éventuellement oxyéthylénés, les esters d'acides gras et de saccharose, les esters d'acides gras polyoxyalkylénés, les alkylpolyglycosides éventuellement oxyalkylénés, les esters d'alkylglucoside, les dérivés de N-alkylglucamine et de N-acylméthylglucamine, les aldobionamides et les oxydes d'amine,
et préférablement parmi les alkylpolyglycosides éventuellement oxyalkylénés.

5. Composition selon l'une quelconque des revendications précédentes, **caractérisée en ce que** le ou les agents tensioactifs amphotères ou zwitterioniques sont choisis parmi les (C₈-C₂₀)-alkylbétaines, les sulfobétaïnes, les (C₈-C₂₀)-alkylamido (C₃-C₈) alkylbétaïnes et les (C₈-C₂₀)-alkylamido(C₆-C₈)alkylsulfobêtaines, préférablement parmi la cocoylbétaïne et la cocamidopropylbétaïne.

6. Composition selon l'une quelconque des revendications précédentes, **caractérisée en ce que** le ou les agents tensioactifs (b) sont choisis parmi les agents tensioactifs amphotères ou zwitterioniques.

7. Composition selon l'une quelconque des revendications précédentes, **caractérisée en ce que** le ou les agents tensioactifs non ioniques, amphotères ou zwitterioniques sont présents selon des proportions d'au moins 0,1% en poids, préférablement allant de 0,1% à 20% en poids et plus préférablement allant de 1% à 15% en poids, par rapport au poids total de la composition.

8. Composition selon l'une quelconque des revendications précédentes, **caractérisée en ce que** les alcools gras ramifiés solides contenant plus de 25 atomes de carbone comprennent de 26 à 60 atomes de carbone et notamment de 26 à 50 atomes de carbone.

9. Composition selon l'une quelconque des revendications précédentes, **caractérisée en ce que** les alcools gras ramifiés solides contenant plus de 25 atomes de carbone sont choisis parmi le 2-dodécylhexadécanol, le 2-tétradécyl-1-octadécanol, le 2-tétradécyl-1-éicosanol, le 2-hexadécyl-1-octadécanol et le 2-hexadécyl-1-éicosanol, et des mélanges de ceux-ci.

10. Composition selon l'une quelconque des revendications précédentes, **caractérisée en ce que** le ou les alcools gras ramifiés solides contenant plus de 25 atomes de carbone sont présents selon une quantité allant de 0,01% à 10% en poids, préférablement de 0,03% à 5% en poids et encore mieux de 0,05% à 3% en poids, par rapport au poids total de la composition.

11. Composition selon l'une quelconque des revendications précédentes, **caractérisée en ce que** le rapport pondéral entre la quantité totale d'agents tensioactifs anioniques (a) et desdits un ou plusieurs agents tensioactifs non ioniques ou amphotères ou zwitterioniques (b), d'une part, et la quantité des un ou plusieurs alcools gras ramifiés solides contenant plus de 25 atomes de carbone, d'autre part, est supérieur ou égal à 2, préférablement allant de 5 à 250 et encore plus préférablement allant de 10 à 100.

12. Composition selon l'une quelconque des revendications précédentes, **caractérisée en ce qu'**elle comprend d'autres ingrédients choisis parmi les silicones, les polymères anioniques, les polymères cationiques, les polymères non ioniques, les polymères amphotères, les épaississants polymères associatifs ou non associatifs, les épaississants non polymères, les agents tensioactifs cationiques, les agents nacrés, les opacifiants, les colorants ou les pigments, les parfums, les huiles minérales, végétales ou synthétiques, les cires y compris les céramides, les vitamines, les agents anti-UV, les agents anti-radicalaires, les agents antipelliculaires, les agents antichute des cheveux, les agents de régénération des cheveux, les agents conservateurs, les stabilisateurs de pH et les solvants, et des mélanges de ceux-ci.

13. Utilisation de la composition selon l'une quelconque des revendications précédentes, pour le lavage, le nettoyage et le conditionnement de matériaux kératiniques, et en particulier de fibres kératiniques.

14. Procédé de lavage, de nettoyage et de conditionnement de matériaux kératiniques, et en particulier de fibres kératiniques, comprenant l'application d'une quantité efficace d'une composition selon l'une quelconque des revendications 1 à 12 auxdits matériaux kératiniques, puis un rinçage après un temps de pose facultatif.
